(19) Europäisches Patentamt
European Patent Office
Office européen des brevets

(11) **EP 2 747 585 B1**

(12) **EUROPEAN PATENT SPECIFICATION**

(45) Date of publication and mention of the grant of the patent:
**22.09.2021 Bulletin 2021/38**

(51) Int Cl.:
*C12N 1/20* [(2006.01)]    *C12R 1/01* [(2006.01)]
*A23C 9/12* [(2006.01)]    *A23L 29/00* [(2016.01)]
*A23L 33/135* [(2016.01)]

(21) Application number: **12758790.5**

(22) Date of filing: **22.08.2012**

(86) International application number:
**PCT/EP2012/066359**

(87) International publication number:
**WO 2013/026886 (28.02.2013 Gazette 2013/09)**

(54) **MICROORGANISMS OF THE SPECIES BACTEROIDES XYLANISOLVENS DSM23964 (CTC1) and their use for the production of a food**

MIKROORGANISMEN DER SPEZIES BACTEROIDES XYLANISOLVENS DSM23964 (CTC1) und ihre Verwendung zur Herstellung eines Lebensmittels

MICROORGANISMES DE L'ESPÈCE BACTEROIDES XYLANISOLVENS DSM23964 (CTC1) et leur utilisation dans un produit alimentaire

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.08.2011 EP 11178319**
         **22.08.2011 US 201161526009 P**

(43) Date of publication of application:
**02.07.2014 Bulletin 2014/27**

(73) Proprietor: **TE Bios Co., Ltd.**
**Chungcheongbuk-do (KR)**

(72) Inventors:
• **GOLETZ, Steffen**
  **13125 Berlin (DE)**
• **ULSEMER, Philippe**
  **13125 Berlin (DE)**
• **TOUTOUNIAN, Kawe**
  **13125 Berlin (DE)**

(74) Representative: **Roth, Carla et al**
**König-Szynka-Tilmann-von Renesse**
**Patentanwälte Partnerschaft mbB**
**Mönchenwerther Straße 11**
**40545 Düsseldorf (DE)**

(56) References cited:
**US-A1- 2004 115 177**    **US-A1- 2010 158 952**
**US-A1- 2011 076 356**    **US-A1- 2011 129 570**

• **DATABASE WPI Week 200978 Thomson Scientific, London, GB; AN 2009-Q64299 XP002717870, & CN 101 560 488 A (UNIV CHINESE AGRICULTURIAL) 21 October 2009 (2009-10-21)**
• **C. MIRANDE ET AL: "Dietary fibre degradation and fermentation by two xylanolytic bacteria Bacteroides xylanisolvens XB1A T and Roseburia intestinalis XB6B4 from the human intestine", JOURNAL OF APPLIED MICROBIOLOGY, 1 January 2010 (2010-01-01), XP055093127, ISSN: 1364-5072, DOI: 10.1111/j.1365-2672.2010.04671.x**
• **CHASSARD ET AL.: "Bacteroides xylanisolvens sp. Nov., a xylan-degrading bacterium isolated from human faeces", INTERNATIONAL JOURNAL OF SYSTEMATIC AND EVOLUTIONARY MICROBIOLOGY, vol. 58, 2008, pages 1008-1013, XP002717871,**
• **P. ULSEMER ET AL: "Safety and tolerance of Bacteroides xylanisolvens DSM 23964 in healthy adults", BENEFICIAL MICROBES, vol. 3, no. 2, 14 March 2012 (2012-03-14), pages 99-111, XP055092809, ISSN: 1876-2883, DOI: 10.3920/BM2011.0051**
• **N. L. ZITOMERSKY ET AL: "Longitudinal Analysis of the Prevalence, Maintenance, and IgA Response to Species of the Order Bacteroidales in the Human Gut", INFECTION AND IMMUNITY, vol. 79, no. 5, 14 March 2011 (2011-03-14), pages 2012-2020, XP055092947, ISSN: 0019-9567, DOI: 10.1128/IAI.01348-10**

EP 2 747 585 B1

• J De Ley ET AL: "The quantitative measurement of DNA hybridization from renaturation rates", European journal of biochemistry / FEBS, 1 January 1970 (1970-01-01), pages 133-142, XP055093106, GERMANY, WEST DOI: 10.1111/j.1432-1033.1970.tb00830.x Retrieved from the Internet: URL:http://www.ncbi.nlm.nih.gov/pubmed/4984993

• None

**Description**

**FIELD OF THE INVENTION**

The present invention relates to food products comprising microorganisms. In particular, food products comprising specific microorganisms of the species *Bacteroides xylanisolvens* are provided. Furthermore, the present invention provides the use of these microorganisms in fermentation.

**BACKGROUND OF THE INVENTION**

Food products containing bacteria as an integral component or production aid are long known in the art. In particular, fermented food such as for example yogurt, cheese, raw sausage, and vegetables that are fermented acidly, are an integral component of our nutrition. Various methods are used for producing fermented foods, wherein for example spontaneous fermentation is used or microorganisms are specifically added to raw food material. Current fermentation methods are in particular based on the addition of starter cultures to the respective raw food material (for example milk to produce yogurt or cheese). Starter cultures afford various benefits as opposed to traditional production processes. For one, economical losses are prevented as a result of fewer defective productions and shortening of production processes. Furthermore, the raw materials can normally react better. As also a mixture of various starter cultures can be utilized, oftentimes the results with respect to taste, safety, and homogeneity, are better. Products can be produced which otherwise would not be possible without targeted intervention in the production process.

An essential objective for developing and improving known fermentation processes is the development and selection of appropriate microorganisms for the fermentation process because they decisively influence the fermentation process. The metabolic activity of the used microorganism is determinative, for example, for the aroma, the acidification degree and/or color of the finished product. Furthermore, microorganisms have a great potential for the field of nutrition when positively affecting the health condition or the metabolism. A known example of fermented foods that positively affect the health of the consumer involves probiotic foodstuffs. A probiotic is a preparation of viable microorganisms which, when consumed in sufficient amounts, have a health-promoting influence on the consumer. Probiotic lactic acid bacteria are used the longest, although yeasts and other species are in use as well.

Most probiotic strains used today are belonging to the genus *Lactobacillus* or *Bifidobacterium,* with only few belonging to other genera like *Enterococcus, Escherichia,* or *Streptococcus.* The main reason is that *Lactobacillus* and *Bifidobacterium* are commonly found in fermented food and therefore being generally recognized as safe for humans. Proposals for the use of nontraditional species in humans generally evoke greater concern about potentially adverse effects. Therefore, current probiotic strains are mainly selected for their mainstream acceptance, a selection process ruling out potential strains with far better health properties.

For example, microorganisms of the genus *Bacteroides* which account for 20 % to up to 40% of the human colon microbiota are not generally used for fermentation or as probiotic strain. This is surprising since certain *Bacteroides* species are known to possess many functions that are beneficial for the human health. *Bacteroides* possess unique metabolic activities involved in the fermentation of carbohydrates, the utilization of nitrogenous substances, and the biotransformation of bile acids and other steroids. Furthermore, *Bacteroides* have been shown to contain certain polysaccharide antigens with immunomodulatory effect and to be strongly involved in the development of the host's immune system and the maintenance of its ability to fight pathogens and diseases.

Microorgansims of the species *Bacteroides xylanisolvens* are described in US 2011/0076356, CN101560488, Mirrande, C. et al. (2010) Journal of Applied Microbiology 109: 451-460, and Chassard, C. et al. (2008) International Journal of Systematic and Evolutionary Microbiology 58: 1008-1013.

However, some *Bacteroides* strains may participate in the development of severe extra-intestinal infections under special conditions. Therefore, some strains are classified as opportunist pathogens and may raise some safety concerns. For example, the *Bacteroides ovartus* microorganisms are not classified in the lowest safety category of microorganisms and thus, have a latent pathogenic risk.

In view of this, it is one object of the present invention to food products comprising microorganisms which fulfill high safety standards and thus, can safely be used for human consumption. In particular, one object is directed towards probiotic food.

**SUMMARY OF THE INVENTION**

The present inventors have found that microorganisms of the species *Bacteroides xylanisolvens* can advantageously be used as food additives or food ingredient in food products, in particular in fermented food and probiotic food. Food products containing microorganisms of the species *Bacteroides xylanisolvens* have a variety of advantageous properties provided by said microorganisms, either used in a viable or in a non-viable form. In particular, these microorganisms have a very low pathogenicity and thus, are highly safe for human consumption, and produce favorable short chain fatty acids.

*Bacteroides xylanisolvens* is a novel species of the genus Bacteroides, which was described for the first time by Chassard et al ("Bacteroides xylanisolvens sp. Nov., a xylan-degrading bacterium isolated from human faeces"; International Journal of Systematic and Evolutionary Microbiology (2008); 58, 1008-1013). It is a xylan-degrading, Gram-negative bacillus bacterium which can be isolated from human feces and thus, is a human commensal microorganism. *Bacteroides xylanisolvens* was deposited as DSM 18836 at the Deutsche Sammlung von Mikroorganismen und Zellkulturen (DSMZ), Inhoffenstraße 7B, 38124 Braunschweig (DE) and is publicly available therefrom. However, its use in food products was not yet known or suggested in the art.

It could be demonstrated that microorganisms of the species *Bacteroides xylanisolvens* have no pathogenicity and thus, can be consumed without a high risk of causing adverse side effects, in particular when consumed by humans. Additionally, due to the low pathogenicity, there are no serious restrictions for the manufacture, distribution and marketing of microorganisms of the species *Bacteroides xylanisolvens* by the statutory regulations, which thus can be fulfilled with little effort. In particular, *Bacteroides xylanisolvens* microorganisms are classified as biological agent of risk group 1 according to the European Directive 2000/54/EC and the German "Biostoffverordnung". Risk group 1 is the lowest of four risk groups and concerns biological agents that are unlikely to cause human disease. Many other microorganisms, also including microorganisms of other Bacteroides species are classified in higher risk groups (e.g. *Bacteroides ovatus* is a biological agent of risk group 2).

Therefore, in a first aspect, the present invention provides a food product comprising a microorganism of the strain DSM 23964 termed CTC1.

In a second aspect, the present invention provides a method for producing a fermented food product wherein a food raw material is combined with a starter culture for fermentation, characterized in that a microorganism of the strain DSM 23964 termed CTC1 is added. Furthermore, a fermented food obtainable by said method is provided.

In a third aspect, the present invention provides a microorganism of the strain DSM 23964 termed CTC1.

As demonstrated in the examples the microorganisms according to the present invention in particular are negative for plasmid DNA material, the most important virulence factors and most of the relevant extracellular enzymes and pathogenic factors, and do not attach to epithelial cells of the human colon. Furthermore, the microorganisms according to the present invention showed no adverse effects in toxicological studies in mice and no adverse effect of any nature in human taking daily doses of up to $8,5*10^{11}$ CTC1 over three weeks. Thus, the microorganisms according to the present invention fulfill a very high safety standard and may be consumed by humans without a risk of unwanted side effects. Furthermore, the microorganisms according to the present invention preferably are sensitive to various antibiotics and thus, may be easily and effectively eliminated, if necessary.

Additionally, it could be demonstrated that the microorganisms according to the invention show a stable and homogeneous cell surface, in particular a stable and homogeneous expression of surface carbohydrate structures which can be used as marker for the product quality. The surface characteristics and in particular its carbohydrate structures can be used as marker for the homogeneity of cultures of microorganisms containing the microorganism according to the present invention, and/or can be used as marker for the amount of microorganisms according to the present invention in a culture.

Moreover, the present inventors have found that the microorganisms according to the present invention are capable of producing short chain fatty acids (SCFAs). Short chain fatty acids (SCFAs) produced as end products of microbial carbohydrate fermentation may have health promoting properties. As an example, propionate was reported to have potential cholesterol reducing effects and anti-lipogenic effects. It may further stimulate satiety and along with acetate and butyrate present an anti-carcinogenic effect.

In a fourth aspect, the present invention provides the use of a microorganism of the strain DSM 23964 termed CTC1 for the production of a food product, in particular as a starter culture for fermentation.

Other objects, features, advantages and aspects of the present invention will become apparent to those skilled in the art from the following description and appended claims.

## DETAILED DESCRIPTION OF THE INVENTION

The present inventors have found that microorganisms of the species *Bacteroides xylanisolvens* are suitable for use in food products, in particular for human consumption. Therefore, in a first aspect, the present invention is directed to a food product comprising a microorganism of the strain DSM 23964 termed CTC1.

A microorganism of the species *Bacteroides xylanisolvens* in particular refers to a microorganism which belongs to the genus Bacteroides and which preferably has one or more of the following characteristics:

a) in a DNA-DNA hybridization assay, it shows a DNA-DNA relatedness of at least 30%, preferably at least 50%, at least 70%, at least 80%, at least 90%, or at least 95%, more preferred at least 98% or at least 99% with the *Bacteroides xylanisolvens* deposited as DSM 18836 or DSM 23964;

b) it displays a level of 16S rRNA gene sequence similarity of at least 95%, preferably at least 97%, at least 98%, or at least 99%, more preferably at least 99,5% with the *Bacteroides xylanisolvens* deposited as DSM 18836 or DSM 23964;

c) it has one or more of the following characteristics:

i) as the *Bacteroides xylanisolvens* deposited as DSM 18836, it is not able to degrade starch;

ii) it has the ability to use and/or metabolize mannitol, in particular D-mannitol, melezitose and/or sorbitol, in particular D-sorbitol, and/or to produce acid from glycerol;

iii) it expresses a glutamyl glutamic acid arylamidase activity;

iv) it is unable to produce indole;

v) it does not show catalase activity;

d) it has one or more of the following characteristics:

i) it is an anaerobic microorganism;

ii) it is non-spore-forming;

iii) it is non-motile; and

iv) it is Gram negative.

Preferably, at least two or at least three, and more preferred all of the above defined criteria a) to d) are fulfilled.

The term "DNA-DNA relatedness" in particularly refers to the percentage similarity of the genomic or entire DNA of two microorganisms as measured by the DNA-DNA hybridization / renaturation assay according to De Ley et al. (1970) Eur. J. Biochem. 12, 133-142 or Huß et al. (1983) Syst. Appl. Microbiol. 4, 184-192. In particular, the DNA-DNA hybridization assay preferably is performed by the DSMZ (Deutsche Sammlung von Mikroorganismen und Zellkulturen GmbH, Braunschweig, Germany) Identification Service. In one embodiment, the DNA-DNA hybridization assay is performed as described in example 1.2, below.

The term "16S rRNA gene sequence similarity" in particular refers to the percentage of identical nucleotides between a region of the nucleic acid sequence of the 16S ribosomal RNA (rRNA) gene of a first microorganism and the corresponding region of the nucleic acid sequence of the 16S rRNA gene of a second microorganism. Preferably, the region comprises at least 100 consecutive nucleotides, more preferably at least 200 consecutive nucleotides, at least 300 consecutive nucleotides or at least 400 consecutive nucleotides, most preferably about 480 consecutive nucleotides. In one embodiment, the region of the nucleic acid sequence of the 16S rRNA gene is flanked by the sequences of SEQ ID NOs: 1 and 2 or their complementary sequences, respectively.

In preferred embodiments, the microorganism of the species *Bacteroides xylanisolvens* stably and/or homogeneously expresses at least one surface carbohydrate structure. Microorganisms stably expressing a surface carbohydrate structure in particular refer to a group of microorganisms wherein at least 50 %, preferably at least 60 %, at least 70 %, at least 80 %, at least 90 %, at least 95 %, at least 97 %, at least 98 %, at least 99 % or about 100 % of the microorganisms express the surface carbohydrate structure. Microorganisms homogeneously expressing a surface carbohydrate structure in particular refer to a group of microorganisms wherein at least 50 %, preferably at least 60 %, at least 70 %, at least 80 %, at least 90 %, at least 95 %, at least 97 %, at least 98 %, at least 99 % or about 100 % of the microorganisms express the surface carbohydrate structure at an expression level which differs from the average expression level by no more than 50 %, preferably no more than 40 %, no more than 30 %, no more than 25 %, no more than 20 %, no more than 15 % or no more than 10 %. A microorganism expressing a surface carbohydrate structure in particular refers to a microorganism which comprises the surface carbohydrate structure in an amount that is detectable by suitable methods as known in the art.

The *Bacteroides xylanisolvens* microorganism preferably is capable of producing short chain fatty acids (SCFAs). Short chain fatty acids (SCFAs) produced as end products of microbial carbohydrate fermentation may have health promoting properties. As an example, propionate was reported to have potential cholesterol reducing effects and anti-lipogenic effects. It may further stimulate satiety and along with acetate and butyrate present an anti-carcinogenic effect. In preferred embodiments, the microorganism is capable of producing one or more SCFAs selected from the group consisting of propionate, acetate, succinate, formate and lactate. Preferably, it is capable of producing propionate and/or acetate. In preferred embodiments, these SCFAs are also present in the food product according to the present invention which contains the microorganism of the strain DSM 23964 termed CTC1. In certain embodiments wherein the food product according to the invention comprises the microorganism of the strain DSM 23964 termed CTC1 in a viable form, said microorganism is still capable of producing said SCFAs inside the body of the consumer after consumption of the food product.

In preferred embodiments, the *Bacteroides xylanisolvens* microorganism is capable of surviving the conditions in, in particular the passage through the stomach, and preferably also the conditions in, in particular the passage through at least a part of the intestine of a human being after ingestion. In particular, surviving the conditions in the stomach of a human being refers to the survival in gastric juice for at least 180 min of at least 50 %, preferably at least 60 %, at least 70 %, at least 80 % or at least 85 % of the *Bacteroides xylanisolvens* microorganisms in a composition comprising the microorganisms, in particular in the food product. Furthermore, surviving the conditions in the intestine of a human being refers to the survival in intestinal juice for at least 240 min of at least 50 %, preferably at least 60 %, at least 70 %, at least 80 %, at least 85 %, at least 90 % or at least 95 % of the *Bacteroides xylanisolvens* microorganisms in a composition comprising the microorganisms, in particular in the food product.

In preferred embodiments, the *Bacteroides xylanisolvens* microorganism has one or more of the following safety characteristics:

(i) it is sensitive to the antibiotics metronidazole, meropenem and/or clindamycin;

(ii) it does not contain the plasmid RP4 (DSM 3876) and/or the plasmid pSC101 (DSM 6202);

(iii) it does not contain the β-lactamase genes cfiA and/or cfxA;

(iv) it does not contain the virulence factors polysaccharide A of *Bacteroides fragilis* and/or enterotoxin Bft of *Bacteroides fragilis* and/or "Ton B-Linked outer membrane protein" encoded by the gene ompW;

(v) it does not show any extracellular DNase activity, extracellular chondroitinase activity, extracellular hyaluronidase activity and/or extracellular neuraminidase activity; and

(vi) it does not attach to epithelial cells of the human colon.

In a preferred embodiment, the *Bacteroides xylanisolvens* microorganism is

a) CTC1, deposited on September 1, 2010 under the accession number DSM 23964 according to the requirements of the Budapest Treaty at the Deutsche Sammlung von Mikroorganismen und Zellkulturen (DSMZ), Inhoffenstraße 7B, 38124 Braunschweig (DE) by the Glycotope GmbH, Robert-Rossle-Str. 10, 13125 Berlin (DE), (DSM 23964),

b) a microorganism derived from CTC1 or

c) a CTC1 homolog.

CTC1 (DSM 23964) belongs to the species *Bacteroides xylanisolvens.* Preferably, CTC1 is a strictly anaerobic, non-spore-forming, non-motile and Gram negative rod-shaped bacterium of 0.4-0.5 μm width and generally 1-2 μm length which grows colonies on Wilkins-Chalgren agar which after 18 h are 2-3 mm in diameter with a circular, milky, raised and convex surface.

A microorganism derived from CTC1 and/or a CTC1 homolog preferably has one or more of the following characteristics:

a) it belongs to the species of *Bacteroides xylanisolvens* as defined above;

b) in a DNA-DNA hybridization assay, it shows a DNA-DNA relatedness of at least 30%, preferably at least 50%, at least 70%, at least 80%, at least 90%, or at least 95%, more preferred at least 98% or at least 99% with CTC1 deposited as DSM 23964;

c) it displays a level of 16S rRNA gene sequence similarity of at least 95%, preferably at least 97%, at least 98%, or at least 99%, more preferably at least 99,5%, and most preferably about 100% with CTC1 deposited as DSM 23964;

d) it is capable of producing short chain fatty acids;

e) it has one or more of the safety characteristics disclosed above; and/or

f) it survives the conditions in the stomach and/or intestine of a human being.

Preferably, at least two, more preferably at least three, at least four or at least five, and most preferred all of the above defined criteria a) to f) are fulfilled.

The term "a microorganism" as used herein may refer to only one unicellular organism as well as to numerous single unicellular organisms. For example, the term "a microorganism of the species *Bacteroides xylanisolvens"* may refer to one single *Bacteroides xylanisolvens* bacterial cell of the species *Bacteroides xylanisolvens* as well as to multiple bacterial cells of the species *Bacteroides xylanisolvens.* The terms "a microorganism of the species *Bacteroides xylanisolvens"* and "a *Bacteroides xylanisolvens* microorganism" are used synonymously herein. In general, the term "a microorganism" refers to numerous cells. In particular, said term refers to at least $10^3$ cells, preferably at least $10^4$ cells, at least $10^5$ or at least $10^6$ cells.

In accordance with the present invention the term "food product" refers to any edible product, in particular any product which can be used for nutrition of humans and/or animals, preferably for human nutrition. Food products may range from isolated nutrients, dietary supplements and specific diets to genetically engineered foods, herbal products, and processed foods such as fermented food, cereals, soups, and beverages. The term food product in particular refers to any nutrient, composition of nutrients or formulation which can be taken orally by a human or animal such as but not limited to nutrients, nutrition additives, food additives, dietary supplements, clinical food, parenteral food, enteral food, food for special dietary use, food of specified health use or functional food that can be applied orally in different forms, such as but not limited to capsules, tablets, emulsions, powder, liquids, as well as in form of any food or drink or as a part of it. In special cases the food product can be given parenterally (parenteral food). The food product can be given by itself or mixed with at least one other ingredient. The food product by itself or its mixture with at least one other ingredient can be given by itself or mixed into a food or a drink. The term food product also means any food, in particular fermented food, beverage, capsule, tablet, emulsion, powder, or liquid. In certain embodiments, the food product provides general health benefits. In particular, the food product may be probiotic.

The food product according to the invention preferably comprises the microorganism of the strain DSM 23964 termed CTC1 in an amount or concentration resulting in a daily dose of about $10^6$ to about $10^{13}$ microorganisms for the consumer. Preferably, the daily dose does not exceed $2.8*10^{12}$ microorganisms, preferably $2.3*10^{12}$ microorganisms, and/or is at least $10^8$ microorganisms, preferably at least $10^9$ microorganisms. In particular, the daily dose is in the range of about $10^{10}$ to about $10^{12}$ microorganisms. The food product preferably is in the form of single unit doses each comprising a daily dose of the microorganism of the strain DSM 23964 termed CTC1 as described above.

The food product according to the invention may contain the microorganism of the strain DSM 23964 termed CTC1 in a viable, non-reproductive, non-viable or lysed form. Preferably, said microorganism is used in a non-pathogenic form, a non-reproductive, non-viable or lysed form.

In a second aspect, the present invention is directed to a method for producing fermented food, wherein a food raw material is combined with a starter culture for fermentation, characterized in that a microorganism of the strain DSM 23964 termed CTC1 is added.

"Fermented food" according to the invention in particular refers to food produced or preserved by the action of microorganisms. In particular, fermentation processes involving the use of bacteria are included. A non-limiting exemplary list of products producible by the method according to the invention includes fermented milk products such as sour whey, curdled milk/sour milk, gelatin, cream cheese, soft cheese, cut cheese, hard cheese, processed cheese, quark, cheese from curdled milk, cooked cheese, kefir, ymer, avran, molasses, yogurt, fruit yogurt, sweet whey, whey butter, fresh cheese, mozzarella, feta cheese, whey powder, sour cream, creme fraiche, mascarpone, smetana, sour cream, sour cream butter, butter, butter oil, semi-fat butter, mildly soured butter, raw sausages such as, for example, salami or salty meat, cacao, coffee, sour dough and soured vegetables such as, for example, sauerkraut. The food raw material is selected in accordance with the fermented food product to be produced.

In the method according to the present invention for producing fermented food, the microorganism of the strain DSM 23964 termed CTC1 may be used as starter culture, either alone or in combination with another microorganism suitable for fermentation. Furthermore, the microorganism of the strain DSM 23964 termed CTC1 may be added to the food raw material, during fermentation or to the fermented food after fermentation. If the microorganism of the strain DSM 23964 termed CTC1 is not used as starter culture, the microorganism of the strain DSM 23964 termed CTC1 preferably is added in a non-reproductive, non-viable or lysed form. If the microorganism of the strain DSM 23964 termed CTC1 is added in a viable form, they may be made incapable of reproduction in the final fermented food. This may be realized for example through irradiation of the microorganisms or heating. Preferably, the microorganism is killed.

The method according to the present invention for producing fermented food preferably comprises at least one of the following method steps:

- inoculating the food raw material with a starter culture which optionally contains a microorganism of the strain DSM 23964 termed CTC1;

- adding at least one sugar, preferably glucose; and

- incubating the raw food material containing the starter culture for fermentation, preferably under anaerobic conditions.

The produced fermented food preferably has one or more of the features described above with respect to the food product according to the present application. It is referred to the above disclosure, features and embodiments of the microorganism of the strain DSM 23964 termed CTC1 and the food product comprising it. The microorganism of the strain DSM 23964 termed CTC1 preferably is added in an amount of at least $10^6$ cells per milliliter raw material, preferably in an amount of about $10^9$ to about $10^{10}$ cells per milliliter raw material, more preferably in an amount of about $1*10^9$ to about $7*10^9$ cells per milliliter raw material.

Furthermore, the present invention provides fermented food obtainable by the method according to the invention for producing fermented food.

In a third aspect, the present invention provides a microorganism of the strain DSM 23964 termed CTC1. The microorganism of the strain CTC1 preferably has one or more of the features described above with respect to the microorganism of the species

*Bacteroides xylanisolvens.*

In preferred embodiments, the microorganism according to the invention has one or more of the following characteristics:

i) as the *Bacteroides xylanisolvens* deposited as DSM 18836, it is not able to degrade starch;

ii) it has the ability to use and/or metabolize mannitol, in particular D-mannitol, melezitose and/or sorbitol, in particular D-sorbitol, and/or to produce acid from glycerol;

iii) it expresses a glutamyl glutamic acid arylamidase activity;

iv) it is unable to produce indole;

v) it does not show catalase activity.

Furthermore, the microorganism according to the invention is a Gram negative, anaerobic microorganism which is non-spore-forming and is non-motile. It preferably is capable of producing short chain fatty acids, in particular as described above, and/or is capable of surviving the conditions in the stomach and optionally the intestine of a human being after ingestion, in particular as described above.

In preferred embodiments, the microorganism according to the invention has one or more of the following safety characteristics:

(i) it is sensitive to the antibiotics metronidazole, meropenem and/or clindamycin;

(ii) it does not contain the plasmid RP4 (DSM 3876) and/or the plasmid pSC101 (DSM 6202);

(iii) it does not contain the β-lactamase genes cfiA and/or cfxA;

(iv) it does not contain the virulence factors polysaccharide A of *Bacteroides fragilis* and/or enterotoxin Bft of *Bacteroides fragilis* and/or "Ton B-Linked outer membrane protein" encoded by the gene ompW;

(v) it does not show any extracellular DNase activity, extracellular chondroitinase activity, extracellular hyaluronidase activity and/or extracellular neuraminidase activity; and

(vi) it does not attach to epithelial cells of the human colon.

The microorganism according to the invention in particular is an isolated microorganism which preferably is not present in its natural environment. In particular, the microorganism is not present inside the human body, preferably it is not present inside a human or animal body. According to one embodiment, the microorganism according to the invention has been isolated from a composition comprising microorganisms which do not belong to the species *Bacteroides xylanisolvens*. According to one embodiment, the microorganism according to the present invention is present in or is obtained from a culture comprising at least 70%, at least 80%, at least 90%, preferably at least 95%, 97%, 99%, most preferred about 100% of microorganisms according to the present invention.

Furthermore, the present invention provides a composition comprising the microorganism according to the present invention. With respect to the characteristics of the microorganism according to the present invention, it is referred to the above disclosure. Preferably, 80% or more, more preferably 85% or more, 90% or more, 95% or more, 98% or more or 99% or more, and most preferably about 100% of the microorganisms in the composition are microorganisms according to the invention. According to one embodiment, the microorganism in the composition is present in a viable, non-reproductive, non-viable or lysed form. According to one embodiment, said composition is a cell culture.

Preferably, the composition comprises at least $10^3$ microorganisms according to the present invention, more preferably at least $10^4$, at least $10^5$ or at least $10^6$ microorganisms according to the present invention.

In a fourth aspect, the present invention provides the use of a microorganism of the strain DSM 23964 termed CTC1 for the production of a food product, in particular as a starter culture for fermentation. With respect to the characteristics of the microorganism of the strain DSM 23964 termed CTC1, it is referred to the above disclosure.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of".

## FIGURES

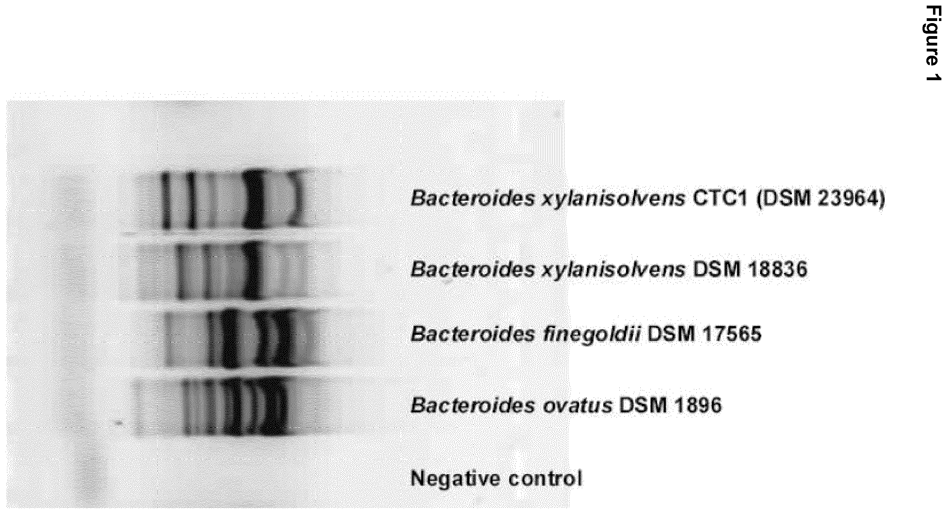

**Figure 1** shows a restriction analysis of the microorganism CTC1 according to the invention and different reference microorganisms. The results support that CTC1 is of the species *Bacteroides xylanisolvens*.

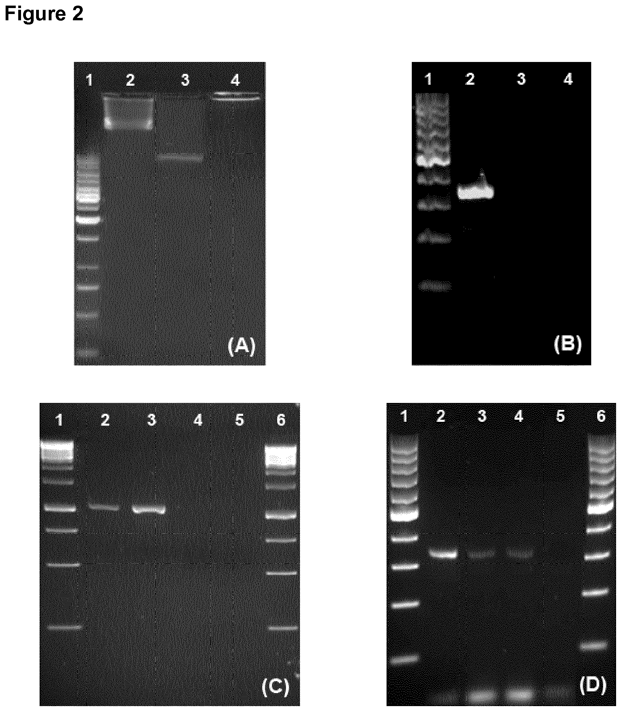

**Figure 2** shows the determination of plasmid and ß-lactamase genes *cfiA, cfxA* and *cepA*. *(A) Plasmid:* 20 μg of each isolated plasmid DNA was loaded. Lanes: 1, 1kb DNA ladder; 2, *E. coli* DSM 3876; 3, *E. coli* DSM 6202; 4, *Bacteroides xylanisolvens* DSM 23964. *(B) cfiA gene:* Lanes: 1, 100bp DNA ladder; 2, *Bacteroides fragilis* TAL 3636; 3, *Bacteroides xylanisolvens* DSM 23964; 4, negative control. *(C) cfxA gene:* Lanes: 1 and 6, 1kb DNA ladder; 2, *Bacteroides ovatus* MN7; 3, *Bacteroides ovatus* MN23; 4, *Bacteroides xylanisolvens* DSM 23964; 5, negative control. *(D) cepA gene:* Lanes: 1 and 6, 100bp DNA ladder; 2, *Bacteroides fragilis DSM* 1396; 3, *Bacteroides ovatus* MN23; 4, *Bacteroides xylanisolvens* DSM 23964; Lane 5, negative control.

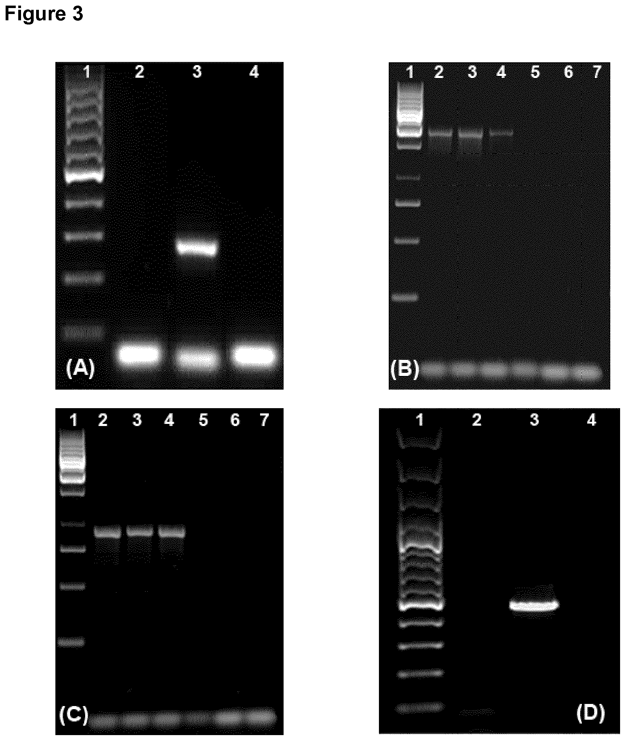

**Figure 3** shows the determination of virulence encoding genes *bft, wcfR, wcfS,* and *ompW*. *(A) bft gene:* Lanes: 1, 100bp DNA ladder; 2, *Bacteroides xylanisolvens* DSM 23964; 3, *Bacteroides fragilis* ATCC 43858; 4, negative control. *(B) wcfR gene:* Lanes: 1, 1kb DNA ladder; 2, *Bacteroides fragilis* DSM 1396; 3, *Bacteroides fragilis* ATCC 43858; 4, *Bacteroides fragilis* DSM 2151; 5, *Bacteroides xylanisolvens* DSM 23964; 6, *Bacteroides fragilis* TAL 3636; 7, negative control. *(C) wcfS gene:* Lanes: Lanes: 1, 1kb DNA ladder; 2, *Bacteroides fragilis* DSM 1396; 3, *Bacteroides fragilis* ATCC 43858; 4, *Bacteroides fragilis* DSM 2151; 5, *Bacteroides xylanisolvens* DSM 23964; 6, *Bacteroides fragilis* TAL 3636; 7, negative control. *(D) ompW gene:* Lanes: 1, 100bp DNA ladder; 2, *Bacteroides xylanisolvens* DSM 23964; 3, *Bacteroides caccae* DSM 19024; 4, negative control.

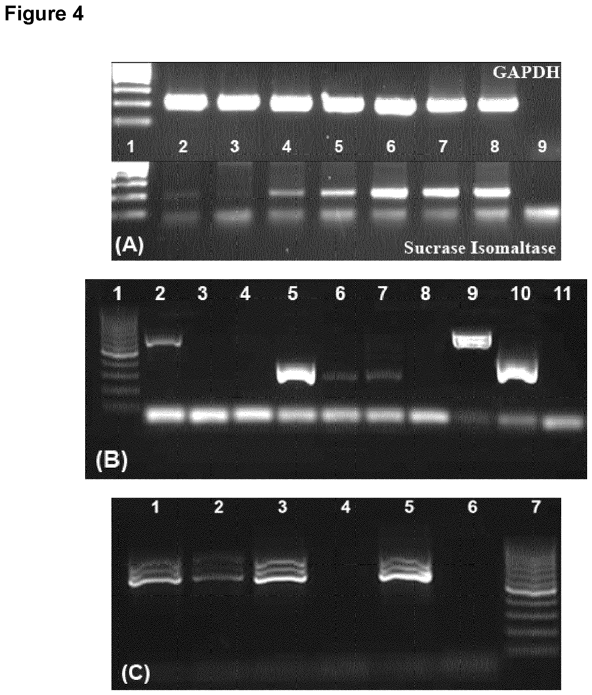

**Figure 4** shows the molecular analysis of the binding of strain DSM 23964 to Caco-2 cells. *(A) GAPDH and sucrose isomaltase.* Lanes: 1, 100 bp DNA ladder (Bioline); 2, $1^{th}$ day; 3, $3^{th}$ day; 4, $6^{th}$ day; 5, $8^{th}$ day; 6, $10^{th}$ day; 7, $13^{th}$ day; 8, $14^{th}$ day, 9, negative control. *(B) Detection of strain DSM* 23964 *and Bacteroides fragilis DSM* 1396. Lanes: 1, 100bp DNA Ladder; 2, strain DSM 23964 after $1^{th}$ wash step; 3, strain DSM 23964 after $6^{th}$ wash step; 4, strain DSM 23964 + Caco-2 cells; 5, *Bacteroides fragilis* DSM 1396 after $1^{th}$ wash step; 6, *Bacteroides fragilis* DSM 1396 after $6^{th}$ wash step; 7, *Bacteroides fragilis* DSM 1396 + Caco-2 cells; 8, Caco-2 cells; 9, Strain DSM 23964; 10, *Bacteroides fragilis* DSM 1396; 11, negative control. *(C) Detection of Lactobacillus acidophilus.* Lanes: 1, *Lactobacillus acidophilus* DSM 9126 after $1^{th}$ wash step; 2, *Lactobacillus acidophilus* DSM 9126 after $6^{th}$ wash step; 3, *Lactobacillus acidophilus* DSM 9126 + Caco-2 cells; 4, Caco-2 cells; 5, *Lactobacillus acidophilus* DSM 9126; 6, negative control; 7, 100bp DNA Ladder.

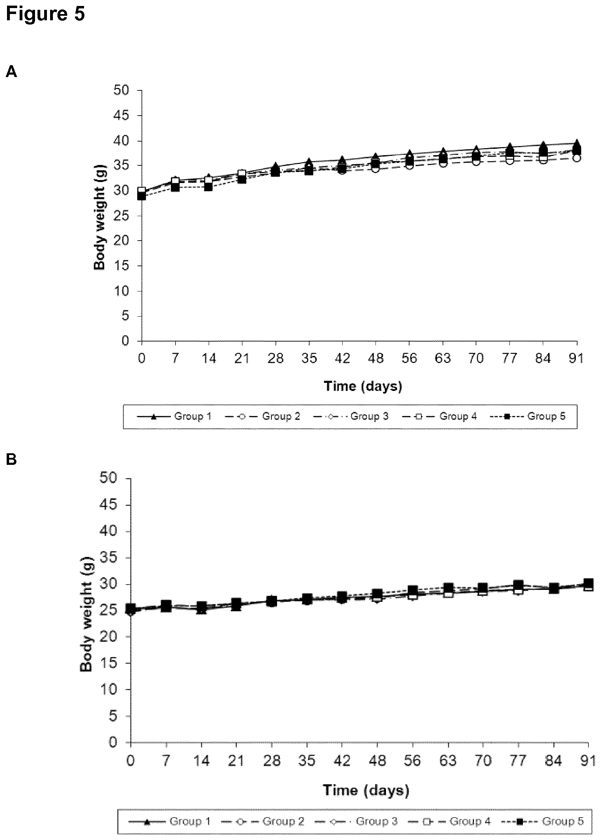

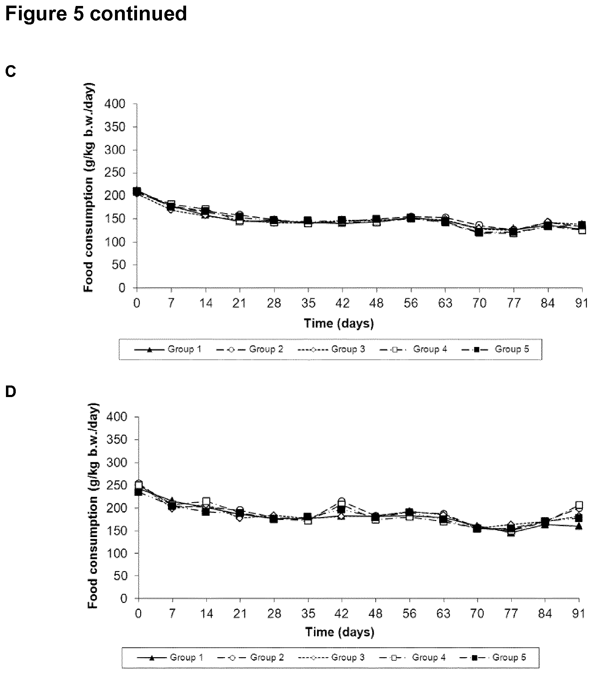

**Figure 5** shows the body weight and food consumption of mice during 90 days oral toxicity study. **(A)** Body weights of male Crl: NMRI mice. **(B)** Body weights of female Crl: NMRI mice. **(C)** Food consumption of male Crl: NMRI mice. **(D)** Food consumption of female Crl: NMRI mice. Mean values per group: Group1 (0), Group2 (1x10^6), Group3 (1x10^7), Group4 (1x10^8) CFU's *Bacteroides xylanisolvens* DSM 23964 and Group5 (1x10^11) *Bacteroides xylanisolvens* DSM 23964 pasteurized / animal / day. Statistical significance indicated by $P \leq 0.01$. Values accorded to Dunnett's test.

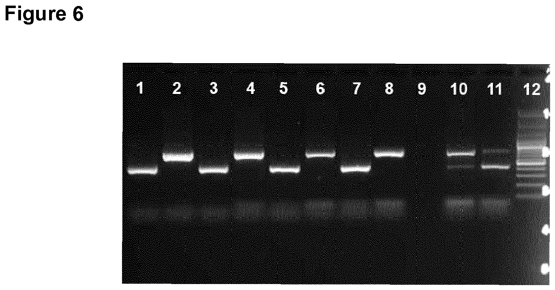

**Figure 6** shows the detection of contamination in injected solutions by multiplex species specific PCR. Lanes: 1, positive control (*Bacteroides xylanisolvens* DSM 23964); 2, Solution 2 (1x10^9 *Bacteroides fragilis* RMA 6791/ml),; 3, Solution 3 (1x10^9 *Bacteroides xylanisolvens* DSM 23964/ml); 4, Solution 4 (1.5x10^8 *Bacteroides fragilis* RMA 6791/ml); 5, Solution 5 (1.5x10^8 *Bacteroides xylanisolvens* DSM 23964/ml); 6, Solution 6 (5x10^6 *Bacteroides fragilis* RMA 6791/ml); 7, Solution 7 (5x10^6 *Bacteroides xylanisolvens* DSM 23964/ml), 8, positive control (*Bacteroides fragilis* RMA 6791); 9, Solution 1 (control group). Contamination controls: Lane 10, mixture of 90% *Bacteroides fragilis* RMA 6791 and 10 % *Bacteroides xylanisolvens* DSM 23964; Lane 11, mixture of 10% *Bacteroides fragilis* RMA 6791 and 90 % *Bacteroides xylanisolvens* DSM 23964. Lane 12, 1 kb DNA Ladder (Fermentas).

**Figure 9** shows the species specific PCR of isolated DNA from punctured abscesses. (**A**) Detection of Bacteroides xylanisolvens in abscesses of animals, which injected with Bacteroides xylanisolvens DSM 23964. Lanes: 1, 100bp DNA Ladder (Fermentas); 2-3, Group 3 (4.6x109 Bacteroides xylanisolvens DSM 23964/kg bw); 4-5, Group 5 (6.9x108 Bacteroides xylanisolvens DSM 23964/kg bw); 6-7, Group 7 (2.3x107 Bacteroides xylanisolvens DSM 23964/kg bw); 8, positive control (Bacteroides xylanisolvens DSM 23964); 9, negative control (water). (**B**) Detection of Bacteroides fragilis in abscesses of animals, which injected with Bacteroides fragilis RMA 6791. Lanes: 1, 100bp DNA Ladder (Fermentas); 2-3, Group 2 (4.6x109 Bacteroides fragilis RMA 6791/kg bw); 4-5, Group 4 (6.9x108 Bacteroides fragilis RMA 6791/kg bw); 6-7, Group 6 (2.3x107 Bacteroides fragilis RMA 6791/kg bw), 8, positive control (Bacteroides fragilis RMA 6791); 9, negative control (Water); 10, 1kb DNA Ladder (fermentas).

## EXAMPLES

**Example 1: *The microorganism according to the invention is a distinct strain of the species Bacteroides xylanisolvens***

*1.1 Taxonomic analysis 1: 16S rRNA sequence similarity*

The 16S rRNA gene sequence (480 bases) of strain CTC1 were amplified by PCR using universal primers 27f (5'-AGAGTTTGATCMTGGCTCAG-3' (SEQ ID NO: 1)) and 519r (5'-GWATTACCGCGGCKGCTG-3' (SEQ ID NO: 2)). PCR products were purified by using the High Pure PCR Product Purification Kit (Roche, Indianapolis, USA) and the DNA concentration and product size estimated by using a Low DNA Mass Ladder (Invitrogen, Carlsbad, USA). PCR products were sequenced using a DYEnamicTM ET Dye Terminator Cycle Sequencing Kit (Amersham Bioscience) and ABI PRISM 3100 capillary sequencer (Applied Biosystems) according to the manufacturer's specifications. The identification of phylogenetic neighbors was initially carried out by the BLAST (Altschul et al., 1997) and megaBLAST (Zhang et al., 2000) programs against the database of type strains with validly published prokaryotic names (Chun et al. 2007). The 50 sequences with the highest scores were then selected for the calculation of pairwise sequence similarity using global alignment algorithm, which was implemented at the EzTaxon server (http://www.eztaxon.org/; Chun et al., 2007). The resulting multiple-sequence alignment was corrected manually by using the program MEGA version 5 (Tamura, 2007) to remove the alignment gaps and ambiguous bases and a phylogenetic tree was constructed according to the neighbor-joining method (Saitou & Nei, 1987) with the program MEGA version 5 (Tamura, 2007).
The 16S rRNA sequence analysis of strain CTC1 showed that this strain clustered with *Bacteroides xylanisolvens* DSM 18836 (100% 16S rRNA sequence similarity), with *Bacteroides ovatus* ATCC 8483 (97,5%), with *Bacteroides thetaiotaomicron* ATCC 29148 (94,2%) and with *Bacteroides finegoldii* DSM 17565 (92,2%). It is generally recognized that similarity values of ≥97% in 16S rRNA gene sequence divergence are significant for species delineation (Stackebrandt & Goebel, 1994). However, Stackebrandt & Ebers (2006) have made the recommendation that this value can be increased to 98.7-99% without sacrificing the quality and precision of a 'species' description, and as an aid to taxonomists.

*1.2 Taxonomic analysis 2: Whole genome DNA-DNA hybridization*

DNA-DNA hybridization is considered the gold standard in taxonomy. The whole genome of the CTC1 strain was submitted to hybridization with the whole genome of *Bacteroides xylanisolvens* DSM 18836, *Bacteroides ovatus* DSM 1896, *Bacteroides thetaiotaomicron* DSM 2079 and *Bacteroides finegoldii* DSM 17565 (those analysis were run at and by the DMSZ (German Collection of Microorganisms and Cell Cultures). Briefly, 3 g biomaterial of each strain to be compared were used for DNA-preparation. Purity of the isolated DNA was analyzed and the DNA was sheared using a French press and denatured at high temperature (100°C, 10 min). The DNA is preferably sheared into fragments having a size of between 200 and 600 kDa, the main fraction being about 450 +/- 100 kDa. Renaturation of the DNA of each strain as well as of a mixture of DNA of both strains in equal concentrations (the final DNA concentrations in the samples is essentially identical and preferably lies between about 20 and 100 μg/ml, in particular about 30 μg/ml) was measured spectrophotometrically using the absorbance at 260 nm. Renaturation was initiated by quickly cooling the solution to a temperature 25°C below the melting temperature of the DNA and the measurements were performed for 30 min. The DNA relatedness was calculated from the different slopes of the renaturation curves of the DNA of each of the single bacterial strain and the mixture of DNA of both strains. In particular, the renaturation rates v' were determined as decrease in absorbance/min (ΔA/t), and the degree of binding (D), i.e. the DNA relatedness, was calculated according to the formula given by De Ley et al. (see above):

$$D = \frac{4\,v'_m - (v'_A + v'_B)}{2\,\sqrt{v'_A\,v'_B}}\,100$$

wherein D is the degree of binding (%), $v'_m$ is the renaturation rate of the mixture, $v'_A$ is the renaturation rate of the DNA of the first strain, and $v'_B$ is the renaturation rate of the DNA of the second strain.

Results of the whole genome hybridisation are shown in Table 1:

**Table 1**

| Reference strain | DNA relatedness to CTC1 |
|---|---|
| *Bacteroides xylanisolvens* DSM 18836 | 98.65 % |
| *Bacteroides ovatus* DSM 1896 | 26.9 % |
| *Bacteroides thetaiotaomicron* DSM 2079 | 28.65 % |
| *Bacteroides finegoldii* DSM 17565 | 25.2 % |

*1.3 Taxonomic analysis 3: Microbiological and biochemical characterization*

The strain DSM 23964 could be identified to be strictly anaerobic, non-spore-forming, non-motile and Gram-negative. The short rods or rod-shaped cells were 0.4-0.5 μm in width and variable in length; generally in the range 1-2 μm. The grown colonies on Wilkins-Chalgren agar (Oxoid) after 18 h were 2 - 3 mm in diameter, with a circular, milky, raised, and convex surface. Initial biochemical analysis showed a 91 % similarity to Bacteroides ovatus species (Databank BioMérieux). In contrast to Bacteroides ovatus, the strain DSM 23964 was unable to utilize starch, to produce indole, and it did not show catalase activity. The biochemical identification of isolated bacteria and their constitutive enzymes and substrate utilization profiles were performed by using rapid ID 32A and API 20A biochemical kits (Biomérieux, Marcy l'Etoile, France) according to the manufacturer's instructions. The results of chemotaxonomic analyses of strain *Bacteroides xylanisolvens* CTC1, *Bacteroides xylanisolvens* DSM 18836, *Bacteroides finegoldii* DSM 17565, *Bacteroides ovatus* DSM 1896, *Bacteroides thetaiotaomicron* DSM 2079 and *Bacteroides fragilis* DSM 1396 are summarized in Table 2. Both strains *Bacteroides xylanisolvens* CTC1 and *Bacteroides xylanisolvens* DSM 18836 had identical biochemical profiles. *Bacteroides xylanisolvens* CTC1 could be differentiated from *Bacteroides ovatus* DSM 1896 by utilization of glycerol, D-sorbitol, D-mannitol and D-melezitose. In addition, *Bacteroides xylanisolvens* CTC1 showed glutamyl glutamic acid arylamidase activity, in contrast to the results for *Bacteroides ovatus* DSM 1896. On the other site, *Bacteroides ovatus* DSM 1896 was able to expressing leucine arylamidase activity, whereas *Bacteroides xylanisolvens* CTC1 did not. Therefore, *Bacteroides xylanisolvens* CTC1 could be differentiated from *Bacteroides finegoldii* DSM 17565, *Bacteroides thetaiotaomicron* DSM 2079 and *Bacteroides fragilis* DSM 1396 (Table 2). In contrast to the results for *Bacteroides xylanisolvens* CTC1 and *Bacteroides xylanisolvens* DSM 18836, *Bacteroides thetaiotaomicron* DSM 2079 showed a large number of positive results in tests for enzyme activities.

**Table 2**

| Biochemical characteristic | 1 | 2 | 3 | 4 | 5 | 6 |
|---|---|---|---|---|---|---|

(continued)

| | 1 | 2 | 3 | 4 | 5 | 6 |
|---|---|---|---|---|---|---|
| *Indole formation* | - | - | - | + | + | - |
| *Enzymatic activities* | | | | | | |
| N- Acetyl- β- Glucosaminidase | + | + | + | - | + | + |
| Glutamic acid Decarboxylase | + | + | + | - | + | + |
| α- Fucosidase | + | + | - | + | + | + |
| Indol production | - | - | - | + | + | + |
| Arginine arylamidase | - | - | - | - | + | - |
| Phenylalanine arylamidase | - | - | - | - | + | + |
| Leucine arylamidase | - | - | - | + | + | + |
| Tyrosine arylamidase | - | - | - | - | + | + |
| Glycine arylamidase | - | - | - | - | + | - |
| Histidine arylamidase | - | - | - | - | + | + |
| Glutamyl glutamic acid arylamidase | + | + | + | - | + | + |
| Serine arylamidase | - | - | - | - | + | - |
| *Acid production from:* | | | | | | |
| D-Mannitol | + | + | - | - | - | - |
| Salicin | + | + | + | + | - | - |
| Esculin hydrolysis | + | + | + | + | + | - |
| Glycerol | + | + | - | - | - | - |
| D-Melezitose | + | + | - | - | - | - |
| D-Sorbitol | + | + | - | - | + | - |
| D-Trehalose | + | + | - | + | + | - |
| *Catalase activities* | - | - | + | + | + | + |

Strains: 1: *Bacteroides xylanisolvens* CTC1; 2: *Bacteroides xylanisolvens* DSM 18836; *3: Bacteroides finegoldii* DSM 17565; 4: *Bacteroides ovatus* DSM 1896; 5: *Bacteroides thetaiotaomicron* DSM 2079; 6: *Bacteroides fragilis* DSM 1396. Characteristics are scored as: '+': positive reaction; '-': negative reaction.

*1.4 Randomly amplified polymorphic DNA (RAPD) pattern and genotype analysis*

To test whether CTC1 is a distinct strain of the species *Bacteroides xylanisolvens,* a RAPD assay was performed. Four different random primers were used in separate reactions (using only one primer in each reaction) for amplification of template DNA. The PCR reaction mixture (50 $\mu$l) contained: Taq Buffer (16 mM $(NH_4)_2SO_4$, 67 mM Tris HCl), 2,5 mM $MgCl_2$, 0.25 mM each dNTP, 1 $\mu$M primer, 2.5 units Taq DNA polymerase and 2 $\mu$l of template DNA. The PCR program was: 95°C for 5 min, 35 cycles of 95°C for 1 min, 50°C for 1 min and 72°C for 1 min, and finally 72°C for 6 min. Band patterns for all primers were analyzed on 1% agarose gels. Thus, for each template DNA, four different band patterns (one for each primer) were obtained.

CTC1 was subjected to the RAPD analysis and the results were compared to that of reference bacteria of the species *Bacteroides xylanisolvens* (DSM 18836), *Baceroides finegoldii* (DSM 17565) and *Bacteroides ovatus* (DSM 1896) (see Fig. 1 for one exemplary primer). The results demonstrate that CTC1 is a distinct strain which is not identical to the known *Bacteroides xylanisolvens* strain DSM 18836.

*1.5 Summary*

The results of the 16S rRNA sequence similarity, the whole genome DNA-DNA hybridization and the biochemical characterization demonstrated that the isolated microorganism CTC1 is of the species *Bacteroides xylanisolvens.* Furthermore, the RAPD analysis showed that CTC1 is a specific and distinct *Bacteroides xylanisolvens* strain which is different from the known *Bacteroides xylanisolvens* strains.

**Example 2: *Effect of simulated gastric juice and intestinal juice***

To simulate the passage through gastrointestinal tract, CTC1 bacteria were submitted to the action of simulated gastric and intestinal juices. The survivor rate for the CTC1 strain in gastric juice was above 90% after 180 min and above 96% after 240 min exposure to intestinal juice.

**Example 3: *Analysis of virulence factors***

*3.1 Antibiotics resistance of CTC1*

The analysis of the minimum inhibitory concentration (MIC) of several antibiotics revealed that the *Bacteroides xylanisolvens* CTC1 strain was resistant to β-lactam drugs like penicillin G, ampicillin and meziocillin. However, it was sensitive to usual antibiotics agents like metronidazole, meropenem and clindamycin and the addition of β-lactamase inhibitor restored the sensitivity to β-lactam drugs.

*3.2 Detection of plasmids in CTC1*

To investigate the potential presence of plasmids in the CTC1 strain, plasmid DNA material was isolated from CTC1 and from both control strains, *E. coli* DSM 3876, and *E. coli* DSM 6202, respectively, which contain the low copy plasmids RP4 (60 kb) and pSC101 (9.4 kb). Measurements of the plasmid DNA concentration at 260 nm revealed no presence of DNA in the plasmid preparation of CTC1. Running the plasmid preparations on an agarose gel confirmed the isolation of both low copy plasmids from the control strains and the absence of detectable plasmid material from *Bacteroides xylanisolvens* CTC1 (Figure 2A).

*3.3 Identification of the β-lactamase genes cfxA, cepA and cfiA in the genome of CTC1*

In order to characterize the β-lactamase activity of the CTC1 strain, specific PCR assays were run for each of the β-lactamase genes cfiA, cfxA, and cepA known for the genus *Bacteroides.* Results indicate that the strain CTC1 exclusively contains the cepA gene (Figure 2B-D).

*3.4 Genes encoding virulence factors in the genus Bacteroides*

The *Bacteroides fragilis* Polysaccharide A (PS A) and the *Bacteroides fragilis* enterotoxin Bft are the most important virulence factors of the genus *Bacteroides.* In order to investigate the presence of the enterotoxin Bft, a specific PCR for the bft gene was performed. In case of PS A, specific PCRs for the highly conserved open reading frames upaY, upaZ, located upstream of the biosynthesis genes of PS A, and for the most important genes wcfR encoding an aminotransferase and wcfS encoding a glycosyltransferase were designed. In contrast to the *Bacteroides fragilis* ATCC 43858, the CTC1 strain does not possess the bft gene. Also the genes wcfR, wcfS and both open reading frames upaY and upaZ could not be detected (Figure 3A-C).

Furthermore, the presence of the gene ompW encoding the virulence factor "Ton B-Linked outer membrane protein", which may be involved in the development of IBD was analyzed. No ompW encoding gene could be detected in the CTC1 strain (Figure 3D).

*3.5 Determination of extracellular enzymes and pathogenic factors of CTC1*

Besides neuraminidase, several strains of the genus *Bacteroides* were described to produce unwanted exoenzymes including collagenase, DNAse and some proteases that may participate in infection processes. The most relevant exoenzyme activities were analyzed by means of PCR (neuraminidase) or enzymatic assays. The CTC1 strain show no DNase, chondroitinase, hyaluronidase, and neuraminidase activities, and only weak β-hemolytic and collagenase activities.

*3.6 Adhesion of CTC1 to Caco-2 cells*

Caco-2 cells were cultivated and differentiation was induced. The results demonstrate that the expression level of GAPDH was constant during differentiation, whereas the level of sucrase isomaltase increased during differentiation. Microscope observation confirmed that the Caco-2 cells were well differentiated as monolayer after 14 days. The binding of bacteria to differentiated Caco-2 cells after 3 hours of co-incubation under anaerobic conditions was analyzed by means of a species-specific PCR performed on supernatants of successive wash steps, and finally on the scraped Caco-2 cells. In contrast to positive controls, CTC1 cells, which of course could be detected in the supernatant of the first wash step, could no longer be detected in later supernatants or on scraped Caco-2 cells, indicating that the CTC1 cells do not attach to epithelial cells of the human colon (Figure 4).

**Example 4: *Toxicological studies***

*4.1 Viability assay*

The viability of *Bacteroides xylanisolvens* CTC1 (DSM 23964) after lyophilization and rehydration was analyzed in several independent experiments. The lowest identified survival rate indicated a minimum concentration of $4\times10^9$ CFU / g viable bacteria. This concentration was accepted as the "available concentration".

*4.2 In vitro mutagenicity study (Ames-Test)*

This test was performed to detect any toxic or mutagenic effects of CTC1 or their fermentation products. Five doses of viable bacteria ranging from 0.28 to 28.5 mg bacteria / plate or one dose of 59 mg pasteurized bacteria / plate were employed in two independent experiments, each carried out with and without metabolic activation. No signs of cytotoxicity and no increase in revertant colony numbers as compared with control counts were observed for any concentration of the 5 test strains with and without metabolic activation, and also in both test formats, plate incorporation and preincubation mode, respectively.

*4.3 In vitro assessment of the clastogenic activity (in vitro chromosomal aberration assay)*

The top concentration of CTC1 employed in the study was 2.8 mg viable bacteria / ml culture medium and 5.9 mg pasteurized bacteria / ml culture medium, which were considered to be the maximum reasonable concentration. In the absence of metabolic activation, the mean incidence of chromosomal aberrations (excluding gaps) observed in the negative control was 1.0 % or 0.5 % after a 4-hour and 24-hour exposure, respectively. None of the concentrations of CTC1, either viable or pasteurized, produced any statistically significant increase in aberrant cells after 4-hour and 24-hour exposure (0.5 % to 2.5 %). In contrast, the positive control presented a 10.5 % and 17.5 % increase in aberrant cells after a 4-hour and 24-hour exposure, respectively. In the presence of metabolic activation, the mean incidence of chromosomal aberrations (excluding gaps) observed in the negative control was 0.5 % after a 4-hour exposure. Again, none of the concentrations of CTC1 either viable or pasteurized produced any statistically significant increase in aberrant cells, resulting in 0.0 % and 1.5 % in two independent experiments, respectively. The positive control presented 13.5 % and 16.5 % aberrant cells after a 4-hour in two experiments, respectively. For all CTC1 concentrations tested, no item-related polyploidy or endoreduplication was noted in the experiments with or without metabolic activation. Furthermore, confirming precedent results, no signs of cytotoxicity were noted at any tested concentration of CTC1 in the experiments with and without metabolic activation.

*4.4 90-Day oral toxicity study in Mice*

The aim of this study was to determine whether the oral intake of CTC1 would have any toxicological effect. Crl: NMRI mice (50 male and 50 female) were allocated to 5 test groups (10 males and 10 females per group) and administrated daily doses of bacteria orally via gavage for 90 days. We tested the effect of $1\times10^6$ to $1\times10^8$ CFU or $1\times10^{11}$ pasteurized CTC1 per animal per day. Results are shown in Figure 5. During the 90 test days no mortality was noted in any group treated with viable or pasteurized CTC1, as in the control group. None of the mice treated or untreated revealed any changes in their behavior or external appearance. Furthermore, the functional observation did not reveal any test item-related influence: motility, faeces consistency, and water consumption, as well as body weight gain and food consumption presented no significant differences throughout the experimental period between the treated groups and with the control group. The hematological examination showed no test item-related influence at any of the tested dose levels of viable and pasteurized CTC1, and no statistically significant differences between the control group and the groups of treated mice was observed. The clinical biochemistry values and the ophthalmological examination revealed no test item-related changes in any group at any dose level for both viable and pasteurized CTC1. Further, macroscopic post-mortem analyses revealed no test item related lesions or abnormalities. Finally, an extensive and detailed histopathological analysis of all organs revealed no differences between the treated groups and with the control group.

*4.5 In vivo pathogenicity of CTC1 (Abscess formation)*

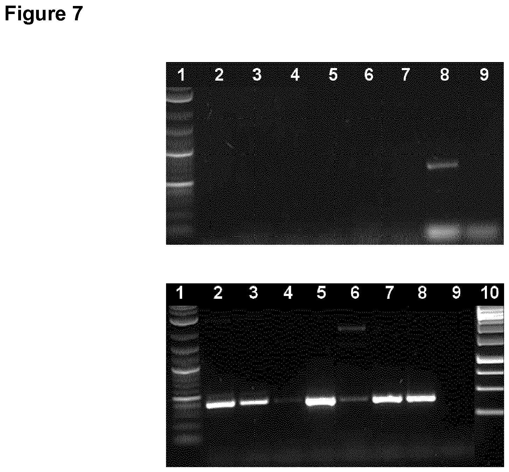

The in vivo intraperitoneal abscess formation model is a well-accepted model to investigate the pathologic properties of opportunistic bacterial strains (McConville et al., 1981; Onderdonk et al. 1984; Thadepalli et al. 2001). Fresh overnight bacterial cultures of *Bacteroides fragilis* RMA 6971 or *Bacteroides xylanisolvens* DSM 23964 (CTC1) were used. A mixture containing $2.3\times10^7$ to $4.6\times10^9$ CFU per kg body weight, 50 % (w/w) autoclaved rat faeces, and 10 % (w/v) barium sulfate was intraperitoneally injected into mice. The viability, bacterial concentration and purity of each item were determined retrospectively after injection on remaining material. In order to identify the presence of *Bacteroides fragilis* and / or *Bacteroides xylanisolvens*, a multiplex species specific PCR as described by Liu et al. (2003) was established. This multiplex PCR also allowed identifying both species in contamination situations where one species would be strongly underrepresented. We confirmed that each single test item contained the wanted bacterial strain and was not contaminated with the other species (see Figure 6). Further potential contaminations were analyzed through plating each test item on appropriate agar and incubated it under aerobic conditions. No single colony could be detected after 48 hours of incubation. In two separate experiments, mice injected with a high dose of CTC1 did not induce the development of more or bigger abscesses as the negative control (Barium sulfate + sterile rat faeces). In contrast, high concentrations of *Bacteroides fragilis* RMA 6971 induced the formation of more and bigger abscesses. After 7 days, 2 abscesses per animal were taken under sterile conditions, the content punctured and submitted to DNA extraction. We evaluated the presence of CTC1 or *Bacteroides fragilis* RMA 6791 in the abscesses by means of species-specific PCRs (Figure 7). *Bacteroides fragilis* RMA 6971 could be detected in all abscesses isolated from groups 2, 4 and 6 injected with $2.3 \times 10^7$ to $4.6 \times 10^9$ *Bacteroides fragilis* per kg body weight, respectively. In contrast, independent of the bacterial concentration injected, CTC1 could not be detected in any of the analyzed abscesses. These results for injected mice with CTC1 clearly indicated that this strain does not induce the formation of abscesses, and that it is actually quickly and completely eradicated by the immune system after i. p. injection.

SEQUENCE LISTING

    <110> Glycotope GmbH

    <120> Microorganisms of the species Bacteroides xylanisolvens

    <130> 53 809 K K

    <150> EP 11178319.7
    <151> 2011-08-22

    <150> US 61/526,009
    <151> 2011-08-22

    <160> 2

    <170> PatentIn version 3.3

    <210> 1
    <211> 20
    <212> DNA
    <213> Artificial

    <220>
    <223> 16S rRNA primer

    <400> 1
    agagtttgat cmtggctcag        20

    <210> 2
    <211> 18
    <212> DNA
    <213> Artificial

    <220>
    <223> 16S rRNA primer

    <400> 2
    gwattaccgc ggckgctg        18

**Claims**

1. A food product comprising a microorganism of the strain DSM 23964 termed CTC1.

2. The food product according to claim 1, comprising the microorganism in an amount or concentration resulting in a daily dose of about $10^6$ to about $10^{13}$ microorganisms for the consumer.

3. The food product according to claim 1 or 2, comprising the microorganism in a viable, non-reproductive, non-viable or lysed form.

4. The food product according to any one of claims 1 to 3, selected from the group consisting of fermented food, probiotic food, functional food, dietary supplements and food additives.

5. A method for producing fermented food, wherein a food raw material is combined with a starter culture for fermentation, **characterized in that** a microorganism of the strain DSM 23964 termed CTC1 is added.

6. The method according to claim 5, having one or more of the following characteristics:

   a) said microorganism is used as starter culture, either alone or in combination with another starter culture;
   b) said microorganism is added to the fermented food during or after fermentation; and/or
   c) said microorganism is used in a viable, non-reproductive, non-viable or lysed form;
   d) said microorganism is added in an amount of at least $10^6$ cells per milliliter raw material, preferably in an amount of about $10^9$ to $10^{10}$ cells per milliliter raw material.

7. The method according to claim 5 or 6, comprising at least one of the following method steps:

   - inoculating the food raw material with a starter culture which optionally contains a microorganism of the strain DSM 23964;
   - adding at least one sugar, preferably glucose; and
   - incubating the raw food material containing the starter culture for fermentation, preferably under anaerobic conditions.

8. Fermented food obtainable by the method according to any one of claims 5 to 7.

9. A microorganism of the strain DSM 23964 termed CTC1.

10. A composition comprising the microorganism according to claim 9.

11. Use of a microorganism of the strain DSM 23964 termed CTC1 for the production of a food product, in particular as a starter culture for fermentation.


**Patentansprüche**

1. Lebensmittelprodukt, enthaltend einen Mikroorganismus des Stammes DSM 23964, bezeichnet als CTC1.

2. Lebensmittelprodukt nach Anspruch 1, umfassend den Mikroorganismus in einer Menge oder Konzentration, die zu einer Tagesdosis von etwa $10^6$ bis etwa $10^{13}$ Mikroorganismen für den Verbraucher führt.

3. Lebensmittelprodukt nach Anspruch 1 oder 2, umfassend den Mikroorganismus in einer lebensfähigen, nicht-reproduktiven, nicht lebensfähigen oder lysierten Form.

4. Lebensmittelprodukt nach einem der Ansprüche 1 bis 3, ausgewählt aus der Gruppe bestehend aus fermentierten Lebensmitteln, probiotischen Lebensmitteln, funktionellen Lebensmitteln, Nahrungsergänzungsmitteln und Lebensmittelzusatzstoffen.

5. Verfahren zur Herstellung eines fermentierten Lebensmittels, wobei ein Lebensmittelrohmaterial mit einer Starterkultur zur Fermentation kombiniert wird, **dadurch gekennzeichnet, dass** ein Mikroorganismus des Stammes DSM 23964, bezeichnet als CTC1, zugegeben wird.

6. Verfahren nach Anspruch 5, mit einem oder mehreren der folgenden Eigenschaften:

a) der genannte Mikroorganismus wird als Starterkultur verwendet, entweder allein oder in Kombination mit einer anderen Starterkultur;
b) der genannte Mikroorganismus wird dem fermentierten Lebensmittel während oder nach der Fermentation zugegeben; und/oder
c) der genannte Mikroorganismus wird in einer lebensfähigen, nicht-reproduktiven, nicht lebensfähigen oder lysierten Form verwendet;
d) der genannte Mikroorganismus wird in einer Menge von mindestens $10^6$ Zellen pro Milliliter Rohmaterial zugesetzt, vorzugsweise in einer Menge von etwa $10^9$ bis $10^{10}$ Zellen pro Milliliter Rohmaterial.

7. Verfahren nach Anspruch 5 oder 6, umfassend mindestens einen der folgenden Verfahrensschritte:

- Beimpfen des Lebensmittelrohmaterials mit einer Starterkultur, die optional einen Mikroorganismus des Stammes DSM 23964 enthält;
- Zugeben von mindestens einem Zucker, vorzugsweise Glukose; und
- Inkubieren des Lebensmittelrohmaterials, das die Starterkultur zur Fermentation enthält, vorzugsweise unter anaeroben Bedingungen.

8. Fermentiertes Lebensmittel, erhältlich nach dem Verfahren gemäß einem der Ansprüche 5 bis 7.

9. Ein Mikroorganismus des Stammes DSM 23964, bezeichnet als CTC1.

10. Zusammensetzung, umfassend den Mikroorganismus nach Anspruch 9.

11. Verwendung eines Mikroorganismus des Stammes DSM 23964, bezeichnet als CTC1, für die Herstellung eines Lebensmittelprodukts, insbesondere als Starterkultur für die Fermentation.


**Revendications**

1. Produit alimentaire comprenant un micro-organisme de la souche DSM 23964 appelé CTC1.

2. Produit alimentaire selon la revendication 1, comprenant le micro-organisme selon une quantité ou une concentration qui conduit à une dose quotidienne d'environ $10^6$ à environ $10^{13}$ micro-organismes pour le consommateur.

3. Produit alimentaire selon la revendication 1 ou 2, comprenant le micro-organisme sous une forme viable, non reproductive, non viable ou lysée.

4. Produit alimentaire selon l'une quelconque des revendications 1 à 3, sélectionné parmi le groupe qui est constitué par les aliments fermentés, les aliments probiotiques, les aliments fonctionnels, les compléments alimentaires et les additifs alimentaires.

5. Procédé pour produire des aliments fermentés, dans lequel une matière brute pour aliments est combinée avec une culture de départ pour la fermentation, **caractérisé en ce qu'**un micro-organisme de la souche DSM 23964 appelé CTC1 est ajouté.

6. Procédé selon la revendication 5, présentant une ou plusieurs des caractéristiques qui suivent :

a) ledit micro-organisme est utilisé en tant que culture de départ, soit seul, soit en combinaison avec une autre culture de départ ;
b) ledit micro-organisme est ajouté aux aliments fermentés pendant ou après fermentation ; et/ou
c) ledit micro-organisme est utilisé sous une forme viable, non reproductive, non viable ou lysée ;
d) ledit micro-organisme est ajouté selon une quantité d'au moins $10^6$ cellules par millilitre de matière brute, de préférence selon une quantité d'environ $10^9$ à $10^{10}$ cellules par millilitre de matière brute.

**7.** Procédé selon la revendication 5 ou 6, comprenant au moins l'une des étapes de procédé qui suivent :

- l'inoculation de la matière brute pour aliments avec une culture de départ qui contient en option un micro-organisme de la souche DSM 23964 ;
- l'ajout d'au moins un sucre, de préférence du glucose ; et
- l'incubation de la matière brute pour aliments qui contient la culture de départ dans le but de sa fermentation, de préférence sous des conditions anaérobies.

**8.** Aliments fermentés pouvant être obtenus au moyen du procédé selon l'une quelconque des revendications 5 à 7.

**9.** Micro-organisme de la souche DSM 23964 appelé CTC1.

**10.** Composition comprenant le micro-organisme selon la revendication 9.

**11.** Utilisation d'un micro-organisme de la souche DSM 23964 appelé CTC1 pour la production de produits alimentaires, en particulier en tant que culture de départ pour la fermentation.

**Figure 1**

# Figure 2

Figure 3

**Figure 4**

# Figure 5

**A**

**B**

# Figure 5 continued

C

D

**Figure 6**

# Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110076356 A
- CN 101560488
- EP 11178319
- US 61526009 B

**Non-patent literature cited in the description**

- **MIRRANDE, C. et al.** *Journal of Applied Microbiology,* 2010, vol. 109, 451-460
- **CHASSARD, C. et al.** *International Journal of Systematic and Evolutionary Microbiology,* 2008, vol. 58, 1008-1013
- **CHASSARD et al.** Bacteroides xylanisolvens sp. Nov., a xylan-degrading bacterium isolated from human faeces. *International Journal of Systematic and Evolutionary Microbiology,* 2008, vol. 58, 1008-1013
- **DE LEY et al.** *Eur. J. Biochem.,* 1970, vol. 12, 133-142
- **HUß et al.** *Syst. Appl. Microbiol.,* 1983, vol. 4, 184-192